# EUROPEAN PATENT APPLICATION

(11) **EP 2 722 780 A1**
(43) Date of publication of application: **23.04.2014**
(21) Application number: 13189225.9
(22) Date of filing: 18.10.2013
(51) Int. Cl.: G06F 19/00, G08B 25/00

(54) **A telemonitoring apparatus for rooms and people**

(30) Priority: 19.10.2012 IT FI20120217
(71) Applicant: Digicsoft S.R.L., 51100 Pistoia (IT)
(72) Inventor: Boarino, Claudio, 22020 Pigra (IT)
(74) Representative: Cinquantini, Bruno

(57) **Abstract**

The present invention relates to a telemonitoring apparatus adapted to monitor at least one person and possibly the room housing her. The invention allows the monitoring of the presence of any emergencies inside the monitored room, such as gas leaks, fire, damage to appliances, etc., but above all the state of health of the person being monitored through the acquisition of a plurality of parameters related to the vital functions of the person herself.

## Description

### Field of the invention

The present invention relates to the technical field of devices and apparatuses for monitoring and remote monitoring of rooms and people (class G06F19 of European patent classification).

### Prior art

The current technology and the increasing spread of equipment capable of being controlled remotely have offered significant new opportunities in the field of remote control and remote assistance. These opportunities have been augmented by the availability of the Internet and its recent huge development.

One area of particular importance that has benefited from the above technologies is the field of remote assistance for the elderly and/or partially disabled people who live alone and may need help and support.

In this area there are products available on the market that offer a simple user interface (typically a button) to the person being monitored through which help can be asked for in case of need.

Other systems of greater complexity involve the construction of networks connected to a plurality of users/patients to be monitored and controlled.

For example, patent application US2006/0010014 describes a system and a method that allow a physician to monitor and manage the health condition of a patient. The system includes a first medical care provider apparatus operated by a doctor and a second programmable remote apparatus that is operated by a patient. Said first medical care provider apparatus develops a script program and then sends the script program to said second programmable remote apparatus through a communication network, such as the World Wide Web. The program script is executed on said second programmable remote apparatus and provides information to the patient about his/her state of health, simultaneously and interactively monitoring the patient's condition, by asking questions to the patient him/herself and getting answers to these questions.

Both types of systems for remote assistance to persons and patients briefly outlined above have a fundamental problem that limits, and can also totally affect, its effectiveness: the need for actions carried out by the person or by the patient being monitored. It is clear that, if the person or the patient being monitored is not able to actuate the emergency button to ask for assistance or is not able to send information relating to his/her state, the monitoring and control systems of the type mentioned above completely lose their effectiveness.

The object of the present invention therefore is to introduce a new system for the remote assistance and remote monitoring of the state of health of a person or a patient.

The system according to the present invention therefore comprises a telemonitoring apparatus adapted to monitor at least one person and possibly the room housing her. The subject system allows the monitoring of the presence of any emergencies inside the monitored room, such as gas leaks, fire, damage to appliances, etc., but above all the state of health of the person being monitored through the acquisition of a plurality of parameters related to the vital functions of the person herself.

### Brief description of the figures

Fig. 1 shows a functional block diagram of a first preferred embodiment of the apparatus according to the present invention.
Fig. 2 shows a functional block diagram of a second preferred embodiment of the apparatus according to the present invention.

### Summary of the invention

The present invention relates to a telemonitoring apparatus adapted to monitor at least one person and possibly the room housing her. The invention allows the monitoring of the presence of any emergencies inside the monitored room, such as gas leaks, fire, damage to appliances, etc., but above all the state of health of the person being monitored through the acquisition of a plurality of parameters related to the vital functions of the person herself.

### Detailed description of the invention

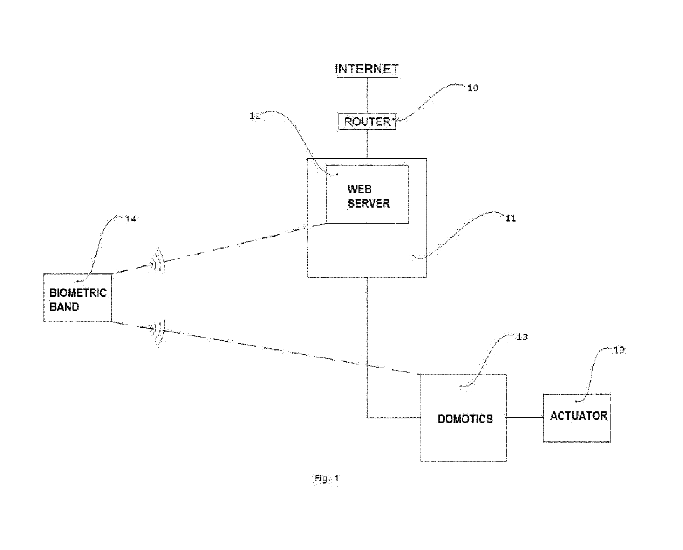

With reference to the accompanying figure 1, a first preferred embodiment of the apparatus according to the present invention comprises standard Internet connection means 10, a management microcomputer 11 - such as the device Fox G20 manufactured by the company *Acmesystems -* provided with its own operating system and provided with a web server 12 associated to said Internet connection means, at least one interface module 13 associated to said microcomputer 11 and adapted to interface with ambient sensors of various types and with commercially available actuating devices, at least one actuating device 19 of the electromechanical type arranged in the room to be monitored and associated to said interface module 13, at least one biometric band 14 for each person to be monitored in the room to be monitored. Said biometric band 14 is preferably made in the shape of a bracelet or wristband, is adapted to be worn by each one of the people to be subjected to monitoring and comprises detection means and transmission means, in wireless mode, of a certain number of parameters linked to the vital functions of the person whereto the biometric band is associated (such as, for example, heart rate, body temperature, movement etc.) and/or a certain number of parameters linked to any specific diseases (such as, for example, the glucose level in the case of people with diabetes to be monitored) through dedicated sensors 17 associated to said biometric band 14. Said biometric band 14 further comprises a button through which, when pressed, the person who is wearing it can send an alert signal through said transmission means with which it is provided. Said biometric band 14 may be such as to connect directly to said microcomputer 11 through one of the commercially available connection protocols or it can connect to the wireless communication interface of said interface module 13.

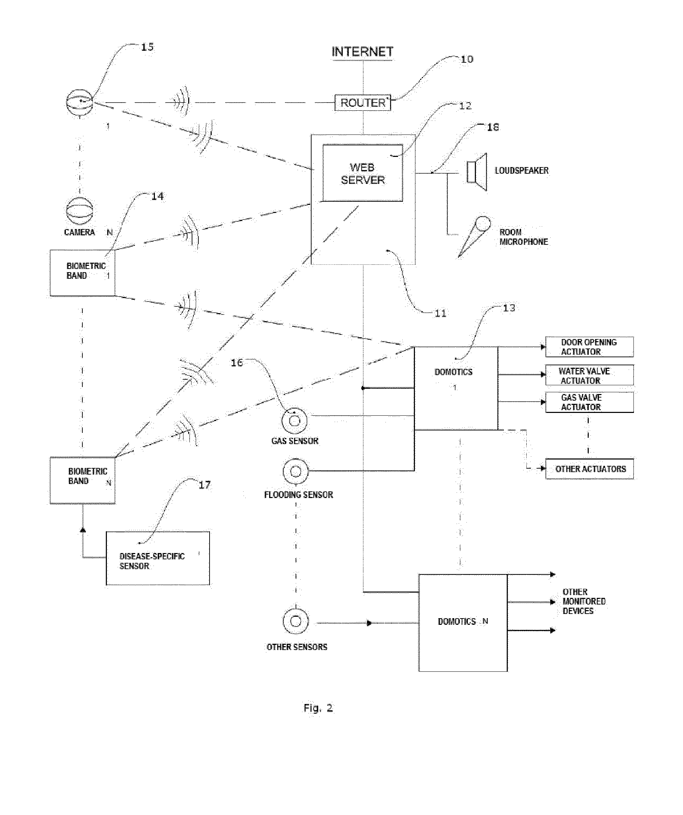

In addition to this basic equipment necessary for the control and remote support of the patient monitored, the present invention may comprise: one or more cameras for the visual observation of the room and for issuing alerts in case of abnormal or suspicious movements; one or more two-directional audio lines to communicate with the people monitored; one or more ambient sensors (temperature, smoke, gas or other) adapted to verify the presence of hazardous situations; one or more additional actuators to remotely remedy the above situations (e.g. solenoid valves for closing gas and water, electro-controlled locks, remote control switches, etc.). With reference to the accompanying Figure 2, therefore, a second preferred embodiment of the apparatus according to the present invention further comprises at least one monitoring camera 15 associated, through a wireless connection, to said web server 12 of microcomputer 11 or directly to said standard Internet connection means 10; at least one ambient sensor 16 associated to said at least one interface module 13 and adapted to monitor ambient parameters such as, for example, temperature, smoke, gas, etc.; at least one two-directional audio line 18 associated to said microcomputer 11.

The operation of the apparatus according to the present invention is as follows: said at least one biometric band 14, said at least one ambient sensor 16 and said at least one monitoring camera 15 gather the biometric parameters of the person being monitored, at least one ambient parameter related to the room where said person is located (temperature, humidity, gas presence, smoke presence, etc.) and the images of said room, respectively; these data are acquired by said interface module 13, which sends them to said management microcomputer 11 which provides, in turn, to process them, identifying any alarm conditions, and to make them available on the World Wide Web through the web interface of said web server 12 for their convenient remote consultation via the Internet.

Any alarm conditions may relate to the fact that the biometric parameters detected by said biometric band 14 and the ambient parameters detected by said at least one ambient sensor 16 are outside the range considered normal or "safety" or that any image recognition software associated to said microcomputer 11 has identified some irregularities in the images captured by said monitoring camera 15.

The apparatus according to the present invention has two distinct operating modes, a so-called "passive" mode and a so-called "active" mode.

In the so-called "passive" mode, the apparatus according to the present invention, on the web interface of said web server, provides a user intended to monitor the room and the person or persons being monitored with the status of the parameters acquired and processed and the images acquired in real time.

The apparatus may advantageously be programmed so as to update the acquisitions made at regular intervals or upon changes in the parameters previously acquired that are significant in percentage.

Said user intended to monitor the room and the person or persons being monitored will therefore have wide access to the data collected (readings of the ambient sensors and measurements of the biometric band), as well as any alerts regarding the fact that there have been no more communications and updates of the parameters monitored in a time interval considered "suspected". This lack of communication may be due to an actual alarm, or to the fact that the person(s) being monitored is/are no longer within the radio range from the equipment, or even to the fact that the battery of the biometric band 14 is low or has failed, etc. The apparatus according to the present invention will therefore allow said user in charge of monitoring to intervene if an abnormal situation is detected, alerting the rescue and facilitating the entrance of the rescuers in the monitored room, for example remotely controlling the entrance door opening by actuating the electromechanical actuating device for opening the door through the interface module and its actuating devices.

In the so-called "active" mode, on the other hand, the apparatus according to the present invention operates automatically, without the need for a supervisor user to decide the actions to take when an emergency condition is detected.

Examples of emergency conditions, in response to which the apparatus according to the present invention must intervene automatically, are: detection of gas, smoke or flooding in the monitored room or the detection of abnormal heartbeat or abnormal body temperature or immobility of the person or persons being monitored.

Upon the occurrence of the above emergency conditions and of other selectable among all those available, given the configuration of the apparatus according to the present invention, said apparatus can react, for example, by sending text messages or phone calls to emergency operators, triggering alarm sirens, automatically opening access to the monitored rooms, etc.

## Claims

1. A telemonitoring apparatus for rooms and people, comprising standard Internet connection means (10); a management microcomputer (11) provided with its operating system and provided with web server (12) associated to said Internet connection means (10); at least one interface module (13) associated to said microcomputer (11) and adapted to interface with ambient sensors and with actuating devices; at least one actuating device (19), arranged in the room to be monitored and associated to said interface module (13); at least one biometric band (14) for each person to be monitored in the room to be monitored, said biometric band (14) being made in the shape of a bracelet or wristband, being adapted to be worn by each one of the people to be subjected to monitoring and comprising detection means and transmission means, in wireless mode, of a certain number of parameters linked to the vital functions of the person to be monitored whereto said biometric band (14) is associated and/or a certain number of parameters linked to any specific diseases through dedicated sensors (17) associated to said biometric band (14).

2. An apparatus according to claim 1, further comprising at least one monitoring camera (15) adapted to shoot and transmit images of said room to be monitored and associated, through a wireless connection, to said web server (12) of said microcomputer (11) or directly to said standard Internet connection means (10); at least one ambient sensor (16) associated to said at least one interface module (13) and adapted to monitor ambient parameters; at least one two-directional audio line (18) associated to said microcomputer (11) and adapted to transmit audio signals from and to said room to be monitored and said people to be monitored.

3. An apparatus according to claims 1 - 2, wherein said microcomputer (11) is adapted to transmit and display, on the web interface of said web server (12), the status of the parameters acquired by said detection means of said biometric band (14).

4. An apparatus according to claims 2-3, wherein said microcomputer (11) is adapted to transmit and provide, on the web interface of said web server (12), the status of the parameters and data acquired by said detection means of said biometric band (14), by said at least one ambient sensor (16), by said at least one monitoring camera (15) and by said at least one two-directional audio line (18).

5. An apparatus according to claims 3-4, wherein said microcomputer (11) comprises means adapted to process the data received by said detection means of said biometric band (14), by said at least one ambient sensor (16), by said at least one monitoring camera (15) and by said at least one two-directional audio line (18) so as to detect any alarm conditions.

6. An apparatus according to claim 5, wherein said microcomputer (11) comprises means adapted to automatically actuate said at least one actuating device (19) based on the occurrence of the detection of any alarm conditions.

7. An apparatus according to claims 1 - 6, wherein said biometric band (14) further comprises a button actuable by the user and adapted to enable the transmission of an alert signal through said transmission means of said biometric band (14).

8. An apparatus according to claims 2-7, wherein said at least one ambient sensor (16) is selected from the group comprising:

9. An apparatus according to claims 1 - 8, wherein said at least one actuating device (19) is electromechanical and is selected from the group comprising: solenoid valves for closing gas and water, electro-controlled locks, remote control switches, auto-diallers.
